# EUROPEAN PATENT APPLICATION

(11) **EP 4 425 505 A1**
(43) Date of publication of application: **04.09.2024**
(21) Application number: 23159226.2
(22) Date of filing: 28.02.2023
(51) Int. Cl.: G16H 40/63, G16H 20/40, A61M 1/14, A61M 1/36

(54) **MEDICAL DEVICE AND METHOD OF CONTROLLING A MEDICAL DEVICE**

(71) Applicant: B. Braun Avitum AG, 34212 Melsungen (DE)
(72) Inventor: NAGY, Tamás Máté, 1222 Budapest (HU); SÁSKA, Dóra, 1112 Budapest (HU)
(74) Representative: Winter, Brandl - Partnerschaft mbB

(57) **Abstract**

The disclosure refers to a medical device comprising regions, components located in the regions, a control unit configured to determine a state of the medical device, determine at least one component of the components that is related to the state of the medical device, and determine display items derived from the determined state of the medical device and concerning the at least one determined component, and a display unit configured to simultaneously display the display items. The display items comprise a detail view area (11) with a representation of the at least one determined component, and an information area (10) with information related to the determined state of the medical device and concerning the at least one determined component. The display items comprise an overall view area (12) with sub-areas (15) which are representations of the regions of the medical device, wherein at least one of the sub-areas (15) is configured to indicate in which of the regions of the medical device the at least one determined component is located. The disclosure also refers to a corresponding method of controlling a medical device.

## Description

The present disclosure relates to a medical device, in particular an extracorporeal blood treatment device, especially a dialysis machine. Further, the present disclosure relates to a method of controlling a medical device, in particular a method of controlling an extracorporeal blood treatment device, especially a method of controlling a dialysis machine.

### Related art

WO 2014 /162000 A1 relates to medical devices with a display, in particular blood treatment machines such as dialysis machines. The medical device may display an alarm message associated to a heparin pump as an example on the display. In addition, this alarm message can be simultaneously displayed on another transparent display integrated into a door in front of the heparin pump to indicate where the heparin pump is located. Such a scheme may facilitate operations of medical device, in particular for a troubleshooting/alarm recognition/problem location. However, the medical device is very customized, which increases the complexity of production and maintenance, and increases the cost of the medical device.

Therefore, it is an urgent need to provide a more general and common solution for a medical device, which can facilitate operations of the medical device and make the operations safer, in particular for an operation for a troubleshooting/alarm recognition/problem location.

### Brief description of the disclosure

The objective of the present disclosure is to eliminate or at least to reduce the disadvantages of the prior art. Specially, a solution according to the present disclosure shall apply to wide and general operating situations for a medical device. The solution according to the present disclosure shall enable to reduce requirements to users, simplify user operations of the medical device, improve assisting and guiding functions for users and/or increase security of operations on the medical device. In particular, alarm recognition and alarm handling indication of the medical device shall be improved, so that the users can speed up the alarm handling process. Especially, the efficiency of the operation shall be improved and/or the possibility of malfunction shall be decreased.

The objective of the present disclosure is solved by a medical device in accordance with claim 1 and by a method of operating a medical device in accordance with claim 10. Advantageous embodiments are claimed in the dependent claims and/or are explained below.

The present disclosure relates to a medical device, especially an extracorporeal blood treatment device, e.g. a dialysis machine, in particular an acute dialysis machine, comprising regions, components located in the regions, a control unit and a display unit.

In particular, the medical device is divided into the regions. The medical device may be divided into the regions based on a spatial distribution of the medical device and/or function blocks of the medical device.

The components described herein may be pumps, valves and/or disposable elements, e.g. one or more infusion sets, one or more fluid circuits, one or more filters, one or more housings, one or more screens, one or more switches, one or more keys/buttons, and/or one or more load cells.

The control unit is configured to determine a state of the medical device (i.e. determined state). In particular, the state of the medical device can be an occlusion of a line, an exceeding of a threshold, especially a pressure or temperature threshold, a position of a movable part of the medical device, an absence or wrong positioning of at least one of the components or an abnormal feedback of at least one of the components. In particular, the control unit may determine the state of the medical device by using sensors, e.g. pressure sensors (in particular arranged for detecting occlusions), position sensors, temperature sensors, flow sensors. Alternatively or additionally, the control unit may determine the state of the medical device by using an input from a user. Therefore, determining may be implemented by receiving signals from at least one sensor or an input unit, comparing the received signals with stored threshold referring to different possible states (in particular, stored in the control unit) and identifying or choosing one of the possible states on the basis of the comparison(s).

The control unit is configured to determine at least one component of the components (i.e. determined component) that is related to the state of the medical device and to determine display items. In particular, each stored and/or determinable state of the medical device is linked to at least one of the components. Therefore, determining the state of the medical device (i.e. identifying or choosing one of the determinable states of the medical device) may lead to determining the at least one component corresponding to the determined state.

The display items are derived from the determined state of the medical device and concern the at least one determined component. In particular, the control unit is configured to derive the display items from the state of the medical device.

The display unit is configured to simultaneously display the display items. The display items comprise a detail view area with a representation of the at least one determined component. Furthermore, the display items comprise an information area with information related to the determined state of the medical device and concerning the at least one determined component.

According to the present disclosure the display items comprise an overall view area with sub-areas which are representations of the regions of the medical device. That is, each of the sub-areas is linked to a corresponding region. At least one of the sub-areas is configured to indicate in which of the regions of the medical device the at least one determined component is located.

The control unit may be a controller, a microcontroller or a computer. The control unit may further comprise a data storage. The data storage may store and access data (e.g., operation processes, operation steps, configuration procedures, tasks, graphical elements, graphical animations, variables, images, text strings, macros), in particular, data for displaying and programs used to implement the methods according to the present disclosure described hereafter. The display unit may include a liquid crystal display, an organic light-emitting diode screen, a touchscreen and/or a cathode ray tube display. The display unit may be configured to display a graphical user interface.

In other words, the display unit is controlled to display a detail view area representing the at least one determined component and at the same time an overall view area indicating the location of the at least one determined component in relation with the whole medical device or at least a major part of the medical device. In this manner, the user's attention may be drawn to the at least one determined component and at the same time the user may be able to identify the location of the at least one determined component in the medical device immediately. Thus, the medical device according to the present disclosure allows that the user can find the at least one determined component in the medical device easily and precisely. The information related to the state of the medical device and the at least one determined component is also displayed simultaneously on the display unit. With the help of this information in combination with the detail view area and the overall view area, the user operation of the medical device can be further simplified and secured. The user experience is therefore remarkably improved. The medical device according to the present disclosure does not require that the users/operators need to be familiar with the medical device. Even if the user is not medical personnel or if the medical device is quite new to the user, the user can still identify the at least one determined component in the medical device efficiently. In a normal operation situation, a sufficient and efficient indication and guide by means of the medical device according to the present disclosure can speed up an operation of the medical device. In an alarm situation, a sufficient and efficient indication and guide by means of the medical device according to the present disclosure can prevent malfunctioning of components and shorten alarm-handling time, therefore can protect the life safety of patients.

Preferably, the information area according to the present application may further comprise information about the determined state of the medical device. The information about the determined state may provide the user with more information to further support the user to operate the medical device correctly, such as a text describing the determined state.

According to an aspect of the present disclosure, the control unit may be configured to determine a next step to be performed according to the determined state of the medical device and the information area may further comprise information of the next step. In particular, the next step may be a step to be performed on the at least one determined component. It can provide the user with more instructions on how to handle the at least one determined component. In combination with the detail view area (regarding a close view of the at least one determined component) and the overall view area (indicating the location of the at least one determined component in relation with the whole or at least a major part of the medical device), it further assists and guides the user to operate the medical device correctly.

According to an aspect of the present disclosure, the control unit may be configured to identify an error according to the determined state of the medical device and the information area may comprise information of the error, e.g. in a form of an alarm. In combination with the detail view area and the overall view area, it further assists and guides the user to perform a trouble shooting efficiently.

According to an aspect of the present disclosure, in addition to the representation of the at least one determined component, the detail view area may further comprise a representation of at least a part of the region of the medical device where the at least one determined component is located and an indicator indicating a position of the representation of the at least one determined component. It allows the user to identify the at least one determined component in the detail view area quickly and clearly.

Preferably, the information area may further comprise an indicator indicating the at least one determined component in conformity with the indicator indicating the at least one determined component in the detail view area.

According to an aspect of the present disclosure, the display items concerning the at least one determined component may be first display items. The at least one determined component may be a first component. The control unit may be configured to determine at least a second component of the components that is also related to the determined state of the medical device. The control unit may be configured to determine second display items derived from the determined state of the medical device and concerning the second component. The display unit may be configured to display simultaneously the first display items and afterwards display simultaneously the second display items.

The detail view area of the first display items may further comprise an indicator indicating a relative position of the second component regarding the first component. The indicator may be a number, a letter, a string, a form (e.g. an arrow) or a character. The indicator may indicate the relative position of the second component regarding the first component for example by showing a direction from the first component to the second component, and/or by arranging the indicator in such a way that a relative position of the indicator regarding the representation of the first component represents the relative position of the second component regarding the first component.

The information area of the first display items may further comprise information related to the determined state of the medical device and concerning the second component. Preferably, the information area of the first and/or second display items may further comprise at least one other indicator indicating the first and/or second component in conformity with the indicator indicating the first and/or second component in the detail view area of the first and/or second display items. Providing the at least one other indicator is advantageous, as it allows to distinguish the representations of more than one determined component (i.e. first and second components) shown in the detail view area, distinguish the information related to the more than one determined components quickly, and match the representations of more than one determined components shown in the detail view area to respective information related to the more than one determined component clearly and quickly. Therefore, the user can identify and operate the more than one determined components more efficiently.

At least one of the sub-areas of the overall view area of the first display items may be configured to indicate in which of the regions of the medical device the second component is located. Preferably, the region where the second component is located is different from the region where the first component is located. Alternatively, it is possible, that the first component and the second component are located in the same region.

According to an aspect of the present disclosure, the second display items may comprise a detail view area with a representation of the second component and an indicator indicating a relative position of the first component regarding the second component. An information area of the second display items may comprise the same information as the information area of the first display items. An overall view area of the second display items may be equal to the overall view area of the first display items. Like the indicator of the first display items, the indicator of the second display items may be a number, a letter, a string, a form (e.g. an arrow) or a character. The indicator of the second display items may indicate the relative position of the first component regarding the second component for example by showing a direction from the second component to the first component, and/or by arranging the indicator in such a way that a relative position of the indicator regarding the representation of the second component represents the relative position of the first component regarding the second component.

Providing first and second display items is suitable for the case that the representation of the first component and the representation of the second component are not appropriate to be displayed simultaneously in the same detail view area on the display unit. In that case, the first display items with the representation of the first component and the second display items with the representation of the second component are displayed subsequently and separately from each other, so that the user can better identify the first and second components respectively. Furthermore, the indicator indicating the relative relation of the second component regarding the first component and the indicator indicating the relative relation of the first component regarding the second component allow the user to better identify a spatial relationship between the first and second components, therefore better identify the first and second components.

According to an aspect of the disclosure, the detail view area may be configured to highlight the representation of the at least one component with a flashlight effect to better draw user's attention to the representation of the at least one component. The flashlight-effect produces a visual effect as if an intense light is focused on a targeted object. The flashlight-effect directs and almost forces the user's attention to the exact component which needs to be paid attention, without being distracted from the residual part of the detail view area, so that the user can identify the at least one determined component which need to be operated/handled/checked.

According an aspect of the disclosure, the flashlight effect may be generated by at least one virtual highlight layer and a virtual dark layer overlapping with each other in the detail view area. Preferably, the at least one highlight layer may comprise a highlight shape, for example a round shape, which is suitable to circle the at least one determined component. Preferably, the highlight layer may be blurred at the edge of the highlight shape.

According to an aspect of the present disclosure, the at least one sub-area may be marked differently from the residual sub-areas. It provides a more direct visual manner to indicate the user the location of the affected component in relation with the whole medical device. For example, the at least one sub-area may be filled with a different color from the residual sub-areas, and/or the at least one sub-area may have different outlines from the residual sub-areas, and/or the at least one sub-area may be shown much brighter or bigger than the residual sub-areas and/or using any animation effect.

A method according to the present disclosure of controlling a medical device comprising regions and components located in the regions comprises steps of:
- determining a state of the medical device;
- determining at least one component of the components that is related to the state of the medical device;
- determining display items derived from the determined state of the medical device and concerning the at least one determined component; and
- displaying simultaneously the display items.

The display items comprise a detail view area with a representation of the at least one determined component. Furthermore, the display items comprise an information area with information related to the determined state of the medical device and concerning the at least one determined component.

The display items comprise an overall view area with sub-areas which are representations of the regions of the medical device. At least one sub-area is configured to indicate in which of the regions of the medical device the at least one determined component is located. In particular, each of the sub-areas is linked to a corresponding region

In particular, the medical device is divided into the regions. The medical device may be divided into the regions based on a spatial distribution of the medical device and/or function blocks of the medical device.

The components described herein may be pumps, valves and/or disposable elements, e.g. one or more infusion sets, one or more fluid circuits, one or more filters, one or more housings, one or more screens, one or more switches, one or more keys/buttons, and/or one or more load cells.

In particular, the state of the medical device can be an occlusion of a line, an exceeding of a threshold, especially a pressure or temperature threshold, a position of a movable part of the medical device, an absence or wrong positioning of at least one of the components or an abnormal feedback of at least one of the components.

Determining the state of the medical device may be implemented by using sensors, e.g. pressure sensors (in particular arranged for detecting occlusions), position sensors, temperature sensors, flow sensors. Alternatively or additionally, Determining the state of the medical device may be implemented by using an input from a user. Therefore, determining may be implemented by receiving signals from at least one sensor or an input unit, comparing the received signals with stored threshold referring to different possible states (in particular, stored in the control unit) and identifying or choosing one of the possible states on the basis of the comparison(s).

In particular, each stored and/or determinable state of the medical device is linked to at least one of the components. Therefore, determining the state of the medical device (i.e. identifying or choosing one of the determinable states of the medical device) may lead to determining the at least one component corresponding to the determined state.

The method according to the present disclosure may comprise a step of storing and/or accessing data (e.g., operation processes, operation steps, configuration procedures, tasks, graphical elements, graphical animations, variables, images, text strings, macros), in particular, data for displaying and programs.

Preferably, the information area according to the present application may further comprise information about the determined state of the medical device.

According to an aspect of the present disclosure, the method may further comprise a step of determining a next step to be performed according to the determined state of the medical device and the information area may comprise information of the next step. In particular, the next step may be a step to be performed on the at least one determined component.

According to an aspect of the present disclosure, the method may further comprises a step of identifying an error according to the determined state of the medical device and the information area comprises information of the error, e.g. in a form of an alarm.

According to an aspect of the present disclosure the display items concerning the at least one determined component may be first display items. The at least one component may be a first component. The method may further comprise the steps of
- determining a second component of the components that is related to the state of the medical device;
- determining second display items derived from the determined state of the medical device and concerning the second component;
- displaying simultaneously the second display items after displaying simultaneously the first display items.

The detail view area of the first display items may further comprise an indicator indicating a relative position of the second component regarding the first component. The indicator may be a number, a letter, a string, a form (e.g. an arrow) or a character. The indicator may indicate the relative position of the second component regarding the first component for example by showing a direction from the first component to the second component, and/or by arranging the indicator in such a way that a relative position of the indicator regarding the representation of the first component represents the relative position of the second component regarding the first component.

The information area of the first display items may further comprise information related to the determined state of the medical device and concerning the second component. At least one of the sub-areas of the overall view area of the first display items may be configured to indicate in which of the regions of the medical device the second component is located. Preferably, the information area of the first and/or second display items may further comprise at least one other indicator indicating the first and/or second component in conformity with the indicator indicating the first and/or second component in the detail view area of the first and/or second display items. Preferably, the region where the second component is located is different from the region where the first component is located. Alternatively, it is possible, that the first component and the second component are located in the same region.

According to an aspect of the present disclosure, the second display items may comprise a detail view area with a representation of the second component and an indicator indicating a relative position of the first component regarding the second component. The second display items may comprise an information area with the same information as the information area of the first display items. The second display items may comprise an overall view area that is equal to the overall view area of the first display items.

Like the indicator of the first display items, the indicator of the second display items may be a number, a letter, a string, a form (e.g. an arrow) or a character. The indicator of the second display items may indicate the relative position of the first component regarding the second component for example by showing a direction from the second component to the first component, and/or by arranging the indicator in such a way that a relative position of the indicator regarding the representation of the second component represents the relative position of the first component regarding the second component.

According to an aspect of the present disclosure the method may further comprises a step of highlighting the at least one component, in the detail view area with a flashlight effect and/or a step of marking the at least one sub-area differently from the residual sub-areas.

In particular, the flashlight effect may be generated by at least one virtual highlight layer and a virtual dark layer overlapping with each other in the detail view area. Preferably, the at least one highlight layer may comprise a highlight shape, for example a round shape, which is suitable to circle the at least one determined component. Preferably, the highlight layer may be blurred at the edge of the highlight shape.

Marking the at least one sub-area differently from another of the sub-areas, may be realized, for example, by filling the at least one sub-area with a different color compared to the residual sub-areas, or outlining the at least one sub-area differently from the residual sub-areas, brightening or amplifying the at least one sub-area compared to the residual sub-areas or using any animation effect. The step of marking the at least one sub-area differently from the residual sub-areas may be executed simultaneously with the step of determining display items.

The method according to the present disclosure in all its variations is advantageous, as an efficient indication of a determined component may be realized.

### Brief description of figures

The disclosure is explained in more detail below using preferred embodiments and referring to the accompanying figures.
Fig. 1 is a schematic view showing a medical device according to the present disclosure;
Fig. 2 is a schematic view showing a display layout on a display unit according to the present disclosure;
Fig. 3 is a view showing a first example of the overall view areas according to the present disclosure;
Fig. 4 is a view showing a second example of the overall view areas according to the present disclosure;
Fig. 5 is a view showing a first example of the detail view area according to the present disclosure;
Fig. 6 is a view showing a second example of the detail view area of Fig. 5 according to the present disclosure with a flashlight-effect;
Fig. 7 is a view showing a flashlight-effect;
Fig. 8 is a schematic view showing virtual layers for generating a flashlight-effect;
Fig. 9 is a first screen shot of display items simultaneously displayed on the display unit according to the present disclosure;
Fig. 10 is a second screen shot of display items simultaneously displayed on the display unit according to the present disclosure;
Fig. 11 is a third screen shot of display items simultaneously displayed on the display unit according to the present disclosure;
Fig. 12 is a fourth screen shot of display items simultaneously displayed on the display unit according to the present disclosure;
Fig. 13 is a flowchart of a method according to the present disclosure.

The figures are schematic in nature and serve only to understand the disclosure. The features of the different embodiments can be interchanged among each other.

### Detailed description of preferred embodiments

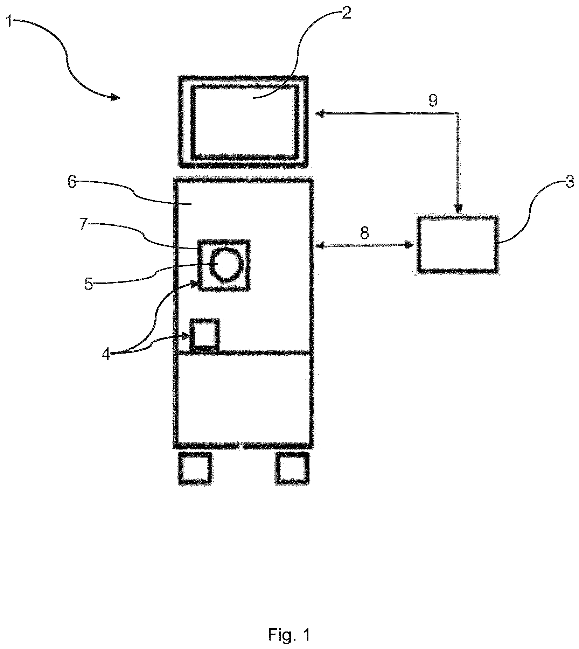

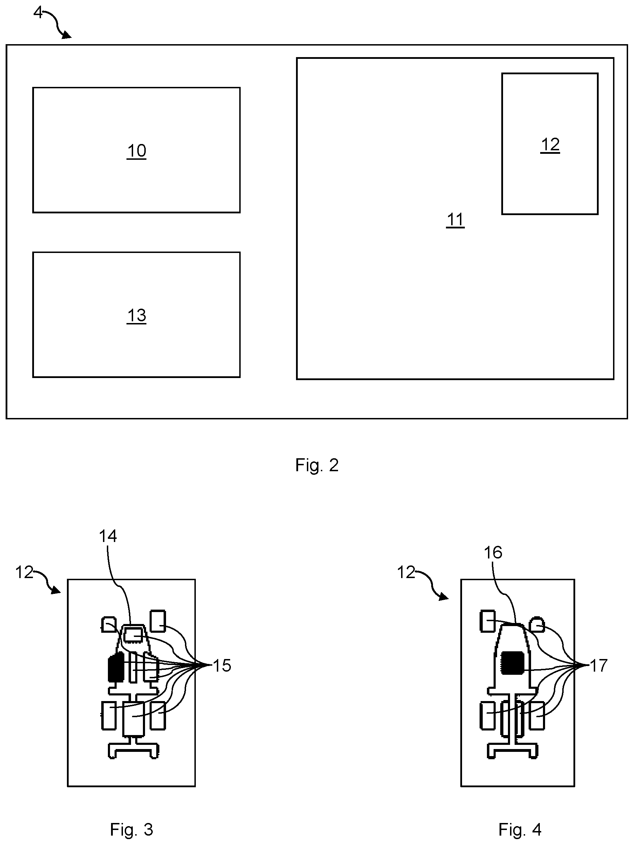

Fig. 1 shows a schematic view of a medical device 1 comprising a display unit 2, a control unit 3, a detection unit 4 and components, e.g. a pump 5, a housing 6 and an infusion set 7. The detection unit 4 is configured to detect a state of the medical device 1. For example, the detection unit 4 may measure a pressure value of the pump 5 by measuring a pressure value of a tube segment of the infusion set 7 before, within or after the pump 5 using a pressure sensor. The detection unit 4 can send a signal 8 indicative of the detected state of the medical device 1 to the control unit 3 and the control unit 3 may receive a signal 8 from the detection unit 4. The control unit 3 is configured to determine at least one component of the components that is related to the detected state of the medical device 1 (also referred as determined component(s)). For example, the control unit 3 may identify that the pressure value of the pump 5 exceeds a predetermined limit value. Based on the detected overpressure on the pump 5, the control unit 3 may further determine that the at least one determined component related to this overpressure is for example the infusion set 7. Further, the control unit 3 is configured to determine display items according to the detected state of the medical device 1 and then control the display unit 2 to display the determined display items. The control unit 3 may send corresponding instructions 9 to the display unit 2. The determined display items will be explained and specified in following figure description regarding Figs 2 to 12.

Fig. 2 shows a schematic view of a display layout on the display unit 2. As shown in Fig. 2, the display items on the display unit 2 comprise an information area 10, a detail view area 11 and an overview area 12. Optionally, a log area 13 may be provided for displaying history information on the display unit 2. The log area 13 may further help the user to track the history information related to a present state (e.g. present alarm). Positions of the areas 10, 11, 12 and 13 shown in Fig. 2 are merely exemplary. The areas 10, 11, 12 and 13 can be arbitrarily arranged in any appropriate positions according to specific application requirements. The areas can overlap, as long as contents of the areas simultaneously shown on the display unit can be seen.

Fig. 3 shows a first example of the overall view area 12 according to the present disclosure. The overall view area 12 represents a whole medical device in sub-areas. Specifically, the overall view area 12 shown in Fig. 3 depicts a front view of a whole of a dialysis machine in a form of an icon 14. The icon 14 is divided into nine sub-areas 15 which represent nine regions of the dialysis machine. In a top portion of the icon 14, there are three sub-areas 15 which depict regions of a citrate load cell, a screen (of the display unit 2) and IV pole from left to right respectively. In a middle portion of the icon 14, there are three sub-areas 15 which depict regions of a blood side kit plate, a filter and a fluid side kit plate from left to right respectively. In a bottom portion of the icon 14, there are three sub-areas 15 which depict regions of fluid bags (e.g. dialysis fluid bag, substitution fluid bag and/or effluent bag). Furthermore, the left sub-area 15 in the middle portion of the icon 14 is displayed in a different color from the residual sub-areas 15 in the icon 14. It indicates that the determined component is located in the region of blood side kit plate and further shows clearly where the region of blood side kit plate is located in relation with the whole dialysis machine.

Fig. 4 shows a second example of the overall view area 12 according to the present disclosure. The overall view area 12 shown in Fig. 4 depicts a rear view of the whole of dialysis machine in a form of an icon16. In contrast to the icon 14, a middle portion of the icon 16 only comprises one sub-area 17 depicting a region of warmer. Further, the sub-area 17 in the middle portion of the icon 16 is displayed in a different color compared to the residual sub-areas 17 in the icon 16. It indicates clearly that the determined component is located in the region of the warmer and indicates that the region of the warmer is located in the back middle portion of the whole dialysis machine.

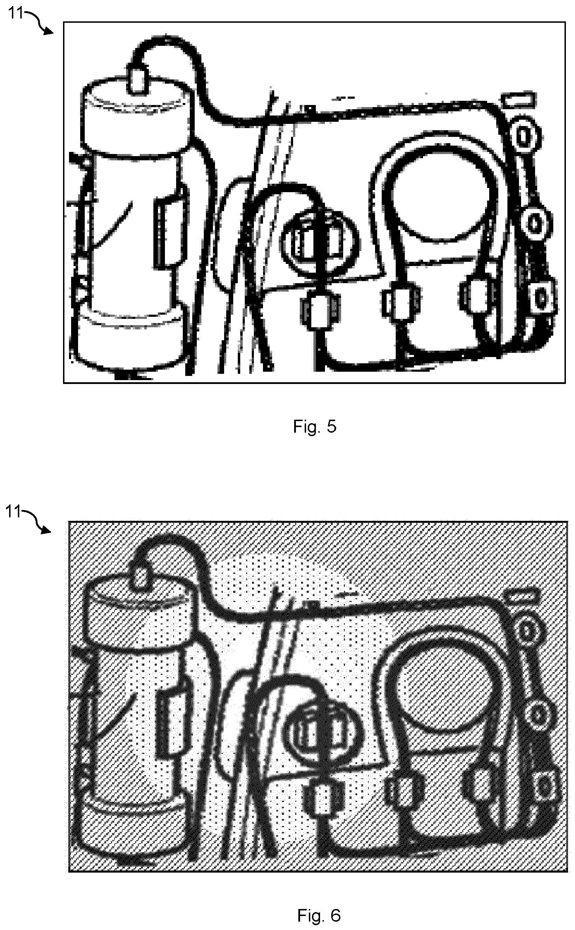

Fig. 5 shows a first example of the detail view area 11. In the first example of the detail view area 11, a partial view of the medical device 1 is shown. The partial view of the medical device 1 is selected and displayed in such a way that the user can see the determined component in the partial view of the medical device 1 easily and clearly.

Fig. 6 shows a second example of the detail view area 11 with a flashlight-effect, in other words, highlight-effect or spotlight effect. It can be seen clearly, compared to the first example of the detail view area 11, a circular zone in the second example of the detail view area 11 is highlighted. Other parts in the second example of the detail view area 11 are shown much darker than the highlighted circular zone. In this manner, the user's attention is directed to the determined components highlighted in circular zone. As shown in Fig. 6, the edge of the circular zone may become dark gradually, so that the user can still identify how the highlighted components in the circular zone connect with other components in a transition zone from light to dark. It gives a user a much more real visual feeling and can further help the user find the determined component in the medical device faster, therefore further improves the user experience.

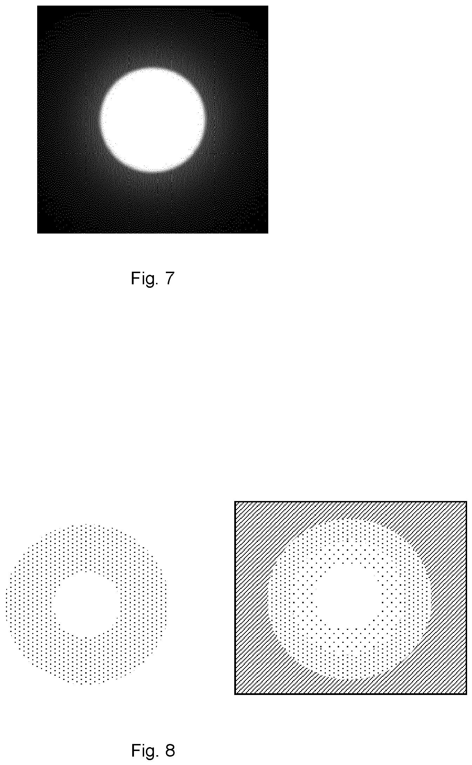

Fig. 7 shows a view of a flashlight-effect in a real visual effect manner. Specifically, the flashlight-effect shown in Fig.7 is achieved by overlapping three virtual layers. The bottom layer is a dark translucent layer. The middle layer is a highlight layer with a highlight circle blurred at 80 pixel. The top layer is a highlight layer with a highlight circle blurred at 3 pixel. The smaller the pixel value at which the highlight circle is blurred is set, the sharper the edge of the highlight circle will turn to dark.

Fig. 8 shows an illustrative view of virtual layers for generating a flashlight-effect. A circle with an annular zone filled with dots on the left side of Fig. 8 indicates a top highlight layer. Compared to the circle on the left side of Fig. 8, a circle with a broader annular zone filled with more densely distributed dots in the middle of Fig. 8 indicates an optional middle highlight layer. The dots indicate dimming of light. A rectangular filled with slashes indicates a dark bottom layer. On the right side of Fig. 8, it shows an integrated view with the top highlight layer, the middle highlight layer and the dark bottom layer. A circular highlight layer may be formed with different radii depending on the size of components that need to be highlighted. A highlight layer may be also formed with other highlight shapes, e.g. geometric shapes other than circles. The blurred edge of the highlight shape of the highlight layer produces a visual effect of light fading gradually. Two highlight layers with blurred edges may produce an even smoother and more natural visual effect of light fading.

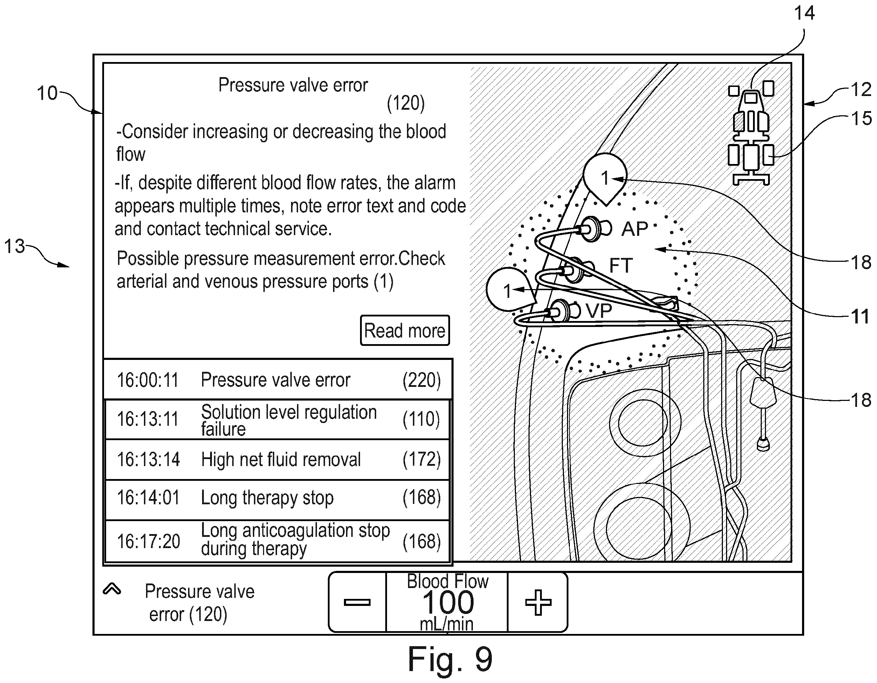

Figs 9 to 12 show several concrete examples of the display items simultaneously displayed on the display unit according to the present disclosure.

Fig. 9 shows a first screen shot of the display items. In Fig. 9, the detail view area 11 is arranged on the right side of the screen shot. In the detail view area 11 of this example, arterial and venous pressure ports are the determined components and are highlighted with a flashlight-effect. Two arrow-like pointers 18 are examples of the indicators indicating a position of the representation of the at least one determined component (in this example arterial and venous pressure ports). The arrow-like pointers 18 can more clearly and more precisely point on the determined components, particularly when more than one determined components shown in the detail view area 11, like in this example. Furthermore, the arrow-like pointers 18 are marked with a number "1" respectively, wherein the number "1" is an example of an indicator indicating the at least one determined component in the detail view area 11. The information area 10 is arranged on the left upper side of the screen shot. The text "Pressure valve error" in the information area 10 corresponds to the determined state in this example, which is also information of an error in this example. The text "- Consider... Check arterial and venous pressure ports (1)" stands for information related to the determined state of the medical device 1 and concerning the at least one determined component and information of the possible next steps. The reference sign "(1)" in the text stands for an example of an indicator indicating the at least one component in conformity with the indicator indicating the at least one component in the detail view area 11 (number "1" in this example). The overall view area 12 is arranged on the right top side of the screen shot. As shown in the overall view area 12 of Fig. 9, the icon 14 representing the dialysis machine is divided into nine sub-areas 15. The left sub-area 15 in the middle portion of the icon 14 is shown in a different color from the residual sub-areas 15 in the icon 14. It indicates that the determined components (in this example arterial and venous pressure ports) are located in the region of blood side kit plate according to the description regarding Fig. 9 and shows where the region of blood side kit plate is located in relation with the whole dialysis machine. Optionally, according to the example, the numbers for arterial and venous pressure ports shown in the detail view area 11 may be numbered differently or be marked in a different way. Accordingly, the reference signs for arterial and venous pressure ports in the information area 10 may be also adapted by using different numbers or showing in different colors. It is especially suitable for the case that alarm handling processes or next operations to be performed on the determined components, e.g. arterial and venous pressure ports in this example are different. In this way, it allows the user to link the determined components and the text regarding the alarm handling processes or next operations clearly and quickly. Furthermore, the log area 13 is arranged on the left lower side of the screen shot. The log area 13 records the log information or history information according to the determined states monitored/detected on the medical device 1.

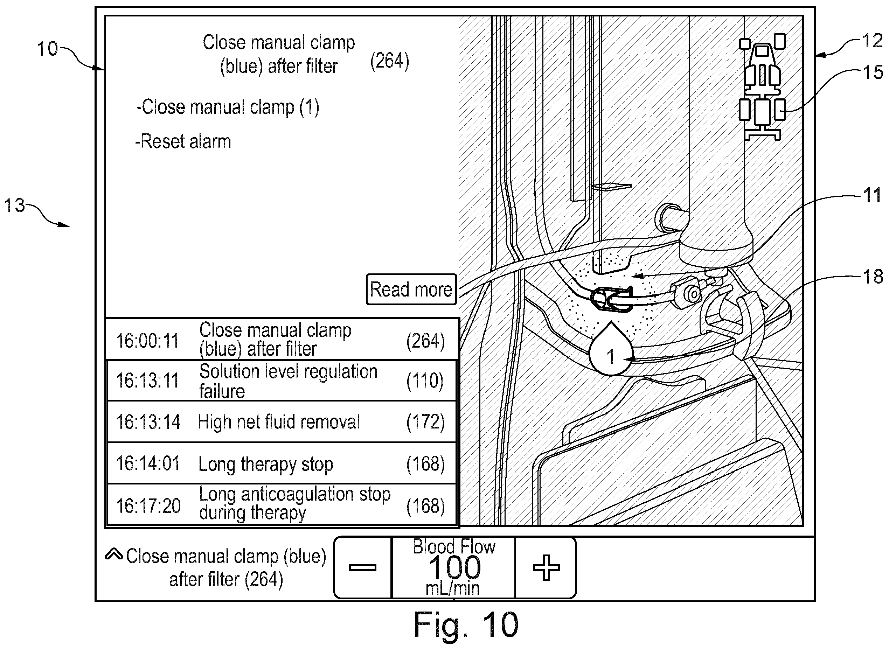

Fig. 10 shows a second screen shot of the display items. In Fig. 10, the detail view area 11 is arranged on the right side of the screen shot. In the detail view area 11 of this example, a clamp is the at least one determined component and is highlighted with a flashlight-effect. The highlight circle for the flashlight-effect in Fig. 10 is smaller than the one in Fig. 9 due to the size of the determined component. The arrow-like pointer 18 is used as an indicator indicating a position of the representation of the at least one component (in this example the clamp). The arrow-like pointer 18 can better and more precisely point on the determined component, especially when the determined component is displayed relative small in the detailed view area 11, like in this example. Furthermore, the arrow-like pointer 18 is marked with a number "1", wherein the number "1" is an example of an indicator indicating the at least one determined component in the detail view area 11. The information area 10 is arranged on the left upper side of the screen shot. The text "Close manual clamp (blue after filter)" in the information area 10 is an example of information of a next step which also implies an error or can be considered as an alarm. The text "- Close manual clamp (1)" stands for information related to the determined state of the medical device 1 (the corresponding state is "the clamp is open") and concerning the at least one determined component (i.e. the clamp) and information of a next step in this example. The reference sign "(1)" in the text stands for an example of an indicator indicating the at least one component in conformity with the indicator indicating the at least one determined component in the detail view area 11 (number "1" in this example), which helps the user to link the operation text regarding the determined component in the information area 10 with the representation of the determined component in the detail view area 11. The overall view area 12 is arranged on the right top side of the screen shot. In the overall view area 12, the icon 14 like in Fig. 3 is shown. In Fig. 10, the middle sub-area 15 in the middle portion of the icon 14 is shown in a different color from the residual sub-areas 15 in the icon 14. It indicates that the determined component (clamp) is located in the region of the filter according to the figure description regarding Fig. 3 and shows where the region of the filter is located in relation with the whole dialysis machine.

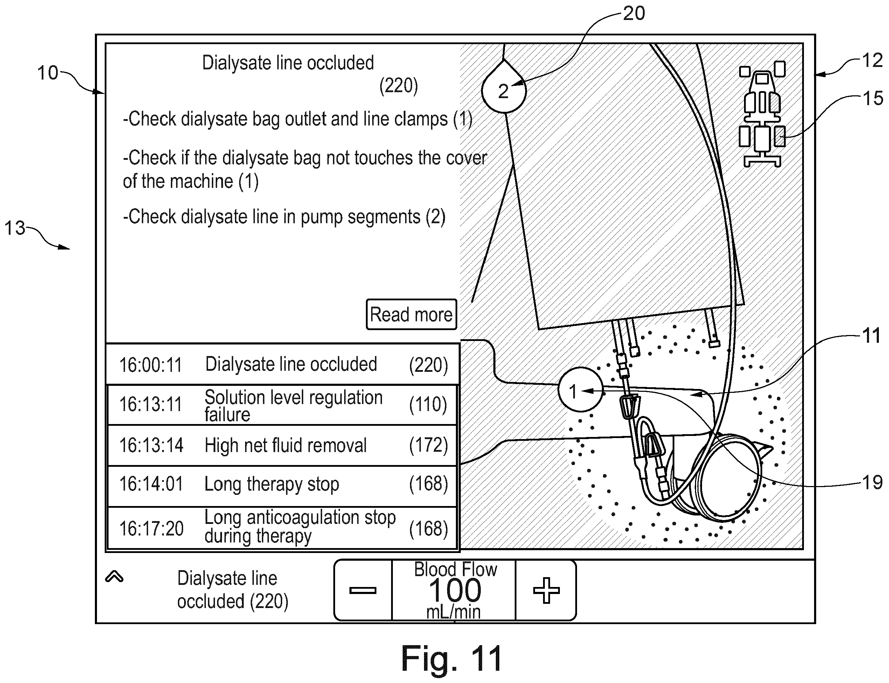

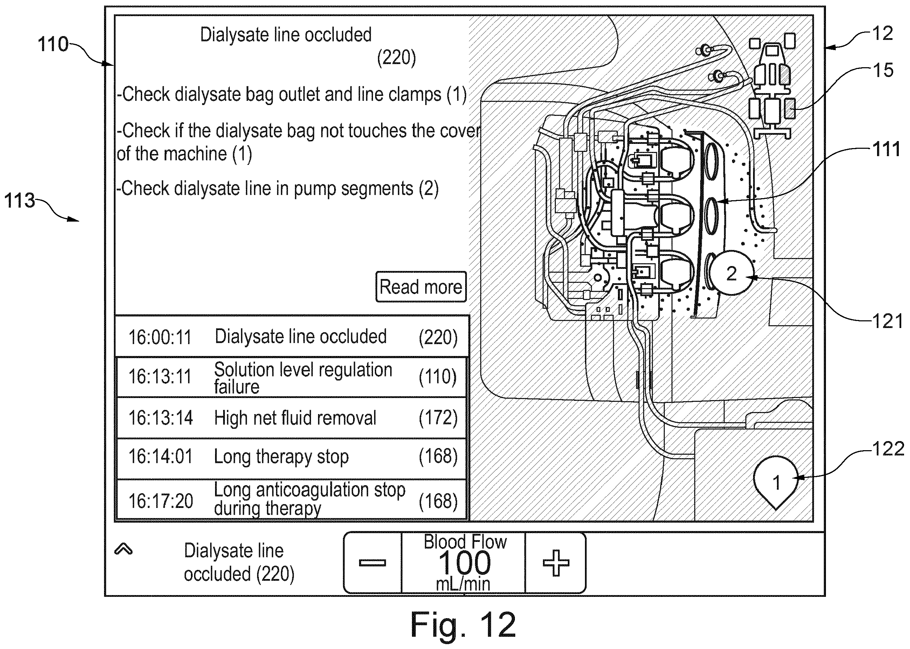

Fig. 11 and Fig. 12 show a third and a fourth screen shot of the display items. Fig. 11 and Fig. 12 together show an example according to an aspect of the disclosure, in which first display items are displayed simultaneously and afterwards second display items are displayed simultaneously. Fig. 11 shows an example of the first display items according to the present disclosure. Fig. 12 shows an example of the second display items according to the present disclosure. Specifically, in this example, dialysate line occlusion is detected. The determined components are dialysate bag outlet, line clamps, covers of the machine and pump segments. The dialysate bag outlet, the line clamps and the covers of the machine are considered as an example of a first component according to the aspect of the disclosure. The pump segments are considered as an example of a second component according to the present disclosure. Accordingly, the state information/information of an error "Dialysate line occluded" is displayed in the information area 10 of Fig. 11 and an information area 110 of Fig. 12. Information related to the determined components and information of next steps "Check dialysate bag outlet....pump segments (2)" are displayed in the information areas 10 and 110 of Figs. 11 and 12. The reference signs "(1)" and "(2)" are examples of indicators indicating the first component (in this example the dialysate bag outlet, the line clamps and the covers of the machine) and the second component (in this example pump segments) in the information areas 10 and 110. The overall view area 12 of Fig. 11 and an overall view area 112 of Fig. 12 both show two sub-areas 15 and 115, respectively, in different color than residual sub-areas. In this example, it indicates that the determined components are located in regions of the fluid side kit plate and the fluid bags (specifically the dialysate bag).

In the detail view area 11 of Fig. 11, a close view of the dialysate bag outlet, the line clamps and the covers of the machine as an example of a representation related to the first component are shown, which are highlighted with a flashlight-effect. A circle pointer 19 is an example of an indicator indicating a position of the representation of the at least one determined component (in this example the dialysate bag outlet, the line clamps and the covers of the machine). An arrow-like pointer 20 indicates an example of an indicator indicating a relative position of the second component (in this example pump segments) regarding the first component (in this example the dialysate bag outlet, the line clamps and the covers of the machine). As shown in the detail view area 11 of Fig. 11, the arrow-like pointer 20 points upwards, which indicates that the second component is above the first component, namely indicates the relative position of the second component regarding the first component. In addition, the pointer 20 itself, regardless of the form with or without an arrow, is located on the top of the representation of the first component, which also indicates that the second component is above the first component, namely indicates the relative position of the second component regarding the first component. The (arrow-like) pointer/indicator 20 provides the user a possibility to orient the second component in the first display items, even though a representation of the second component (pump segments) is not visible in this example shown in Fig. 11. Numbers "1" and "2" in the circle pointer 19 and the arrow-like pointer 20 respectively are examples of indicators indicating the first component and the second component in the detail view area 11. The numbers "1" and "2" are in conformity with the reference signs "(1)" and "(2)" respectively, which can help the user quickly link/associate text regarding the first component in the information area 10 to the representation of the first component in the detail view area 11 and link/associate text regarding the second component in the information area 10 to the representation of the second component in the detail view area 11.

In a detail view area 111 of Fig. 12, a close view of the pump segments as an example of a representation related to the second component is shown, which is highlighted with a flashlight-effect. A circle pointer 121 is an example of an indicator indicating a position at which the representation of the at least one component is located (in this example the pump segments). An arrow-like indicator 122 indicating a relative position of the first component (the dialysate bag outlet, the line clamps and the covers of the machine) regarding the second component (pump segments). As shown in the detail view area 111 of Fig. 12, the arrow-like pointer 122 points downwards, which indicates that the first component is below the second component, namely indicates the relative position of the first component regarding the second component. In addition, the pointer 122 itself, regardless of the form with or without an arrow, is located on the bottom of the representation of the second component, which also indicates that the first component is below the second component, namely indicates the relative position of the first component regarding the second component. The (arrow-like) pointer/indicator 122 provides the user a possibility to orient the first component in the second display items, even though a representation of the first component is not visible in this example shown in Fig. 12.

The detail view areas of Figs. 11 and 12 use different images, in order to allow users to better identify the determined components (first and second components) in the detail view areas 11 and 111. The different indicators such as the arrow-like pointers, circle pointers, numbers and reference signs shown in Figs. 11 and 12 further help the user identify the affected components and their locations in the medical device 1 even more quickly and without high demand of the medical experience to the user, and further help the user perform corresponding operations based on text displayed in the information area 10 and 110 correctly.

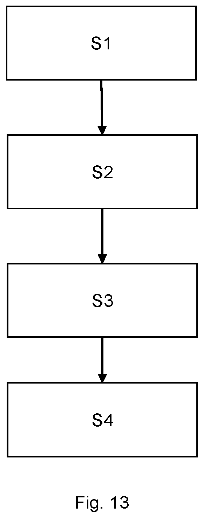

Fig. 13 shows a flowchart of a method according to the present disclosure. The method comprises a step S1 of determining a state of the medical device 1, a step S2 of determining at least one component of the components that is related to the determined state of the medical device 1, a step S3 of determining display items derived from the determined state of the medical device 1 and concerning the at least one determined component and a step S4 of displaying simultaneously the display items. The display items comprise:
a detail view area 11 comprising a representation of the at least one component,
an information area 10 comprising information related to at least one component, and
an overall view area 12 comprising sub-areas 15 which are representations of the regions of the medical device 1, wherein at least one sub-area 15 is configured to indicate in which of the regions of the medical device 1 the at least one component is located.

The step 1 (S1) may be triggered by a measurement and/or an input, in particular a manual input, from a user. In an alarm situation, the step 2 (S2) may be triggered by detecting an abnormal value detected by the detection unit 4. The steps 3 and 4 (S3 and S4) aim to allow the user to identify the at least one component according to the present disclosure quickly and precisely. The targeted defined display items displaying simultaneously on the display unit can guide the user to find the determined component quickly, inform the user about error and enable the user to operate the determined component correctly, therefore the method according to the present disclosure remarkably shortens troubleshooting time.

### List of reference signs

- 1: medical device
- 2: display unit
- 3: control unit
- 4: detection unit
- 5: pump
- 6: housing
- 7: infusion set
- 8: signal
- 9: instruction
- 10; 110: information area
- 11; 111: detail view area
- 12; 112: overall view area
- 13; 113: log area
- 14: icon depicting a front view of a dialysis machine
- 15; 115: sub-area regarding the front view of the dialysis machine
- 16: icon depicting a rear view of the dialysis machine
- 17: sub-area regarding the rear view of the dialysis machine
- 18, 19; 121: pointer/indicator indicating a position of the representation of the at least one determined component
- 20; 122: pointer/indicator indicating a relative position of the second component regarding the first component

## Claims

1. Medical device (1), especially an extracorporeal blood treatment device, comprising
regions,
components located in the regions,
a control unit (3) configured to determine a state of the medical device (1), determine at least one component of the components that is related to the state of the medical device (1), and determine display items derived from the determined state of the medical device (1) and concerning the at least one determined component, and
a display unit (2) configured to simultaneously display the display items, the display items comprising:
a detail view area (11; 111) comprising a representation of the at least one determined component, and
an information area (10; 110) comprising information related to the determined state of the medical device (1) and concerning the at least one determined component,
**characterized in that**
the display items comprise an overall view area (12; 112) with sub-areas (15; 115) which are representations of the regions of the medical device (1), wherein at least one of the sub-areas (15; 115) is configured to indicate in which of the regions of the medical device (1) the at least one determined component is located.

2. Medical device (1) according to claim 1, wherein the control unit (3) is configured to determine a next step to be performed according to the determined state of the medical device (1) and the information area (10; 110) further comprises information of the next step.

3. Medical device (1) according to any one of claims 1 or 2, wherein the control unit (3) is configured to identify an error according to the determined state of the medical device (1) and the information area (10; 110) comprises information of the error.

4. Medical device (1) according to any one of claims 1 to 3, wherein, in addition to the representation of the at least one determined component, the detail view area (11; 111) further comprises a representation of at least a part of the region of the medical device (1) where the at least one determined component is located and an indicator (18, 19; 121) indicating a position of the representation of the at least one determined component.

5. Medical device (1) according to any one of claims 1 to 4, wherein
the display items concerning the at least one determined component are first display items;
the at least one determined component is a first component;
the control unit (3) is configured to determine at least a second component of the components that is also related to the determined state of the medical device (1) and determine second display items derived from the determined state of the medical device (1) and concerning the second component; and
the display unit (2) is configured to display simultaneously the first display items and afterwards display simultaneously the second display items, wherein
the detail view area (11) of the first display items further comprises an indicator (20) indicating a relative position of the second component regarding the first component;
the information area (10) of the first display items further comprises information related to the determined state of the medical device (1) and concerning the second component; and
at least one of the sub-areas (15) of the overall view area (12) of the first display items is configured to indicate in which of the regions of the medical device (1) the second component is located.

6. Medical device (1) according to claim 5, wherein the second display items comprise:
a detail view area (111) comprising a representation of the second component and an indicator (122) indicating a relative position of the first component regarding the second component;
an information area (110) that comprises the same information as the information area (10) of the first display items; and
an overall view area (112) that is equal to the overall view area (12) of the first display items.

7. Medical device (1) according to any one of claims 1 to 6, wherein the detail view area (11; 111) is configured to highlight the representation of the at least one component with a flashlight effect.

8. Medical device (1) according to claim 7, wherein the flashlight effect is generated by at least one virtual highlight layer and a virtual dark layer overlapping with each other in the detail view area.

9. Medical device (1) according to any one of claims 1 to 8, wherein the at least one sub-area (15; 115) is marked differently from the residual sub-areas.

10. Method of controlling a medical device (1) comprising regions and components located in the regions, comprising steps of:
- determining a state of the medical device (1);
- determining at least one component of the components that is related to the determined state of the medical device (1);
- determining display items derived from the determined state of the medical device (1) and concerning the at least one determined component; and
- displaying simultaneously the display items, wherein
the display items comprise:
a detail view area (11; 111) comprising a representation of the at least one determined component; and
an information area (10; 110) comprising information related to the determined state of the medical device (1) and concerning the at least one determined component;
**characterized in that**
the display items comprise an overall view area (12; 112) with sub-areas (15; 115) which are representations of the regions of the medical device (1), wherein at least one sub-area (15; 115) is configured to indicate in which of the regions of the medical device (1) the at least one determined component is located.

11. Method according to claim 10, wherein the method further comprises a step of determining a next step to be performed according to the determined state of the medical device (1) and the information area (10; 110) comprises information of the next step.

12. Method according to claim 10 or 11, wherein the method further comprises a step of identifying an error according to the determined state of the medical device (1) and the information area (10; 110) comprises information of the error.

13. Method according to any one of claims 10 to 12, wherein
the display items concerning the at least one determined component are first display items;
the at least one component is a first component;
the method further comprises the steps of
- determining a second component of the components that is related to the state of the medical device (1);
- determining second display items derived from the determined state of the medical device (1) and concerning the second component;
- displaying simultaneously the second display items after displaying simultaneously the first display items; wherein
the detail view area (11) of the first display items further comprises an indicator (20) indicating a relative position of the second component regarding the first component;
the information area (10) of the first display items further comprises information related to the determined state of the medical device (1) and concerning the second component; and
at least one of the sub-areas (15) of the overall view area (12) of the first display items is configured to indicate in which of the regions of the medical device (1) the second component is located.

14. Method according to claim 13, wherein the second display items comprise:
a detail view area (111) comprising a representation of the second component and an indicator (122) indicating a relative position of the first component regarding the second component;
an information area (110) that comprises the same information as the information area (10) of the first display items; and
an overall view area (112) that is equal to the overall view area (12) of the first display items.

15. Method according to any one of claims 10 to 14, wherein the method further comprises a step of highlighting the at least one component in the detail view area with a flashlight effect and/or a step of marking the at least one sub-area differently from the residual sub-areas.
